# EUROPEAN PATENT APPLICATION

(11) **EP 3 945 134 A1**
(43) Date of publication of application: **02.02.2022**
(21) Application number: 21175929.5
(22) Date of filing: 26.05.2021
(51) Int. Cl.: C12N 7/00

(54) **ADENOVIRUS-ASSOCIATED VIRUSES SEPARATION METHOD**

(30) Priority: 29.07.2020 US 202016942156
(71) Applicant: Pall Corporation, Port Washington, NY 11050 (US)
(72) Inventor: HEJMOWSKI, Adam, Clinton, MA 01510 (US)
(74) Representative: Hoeger, Stellrecht & Partner Patentanwälte mbB

(57) **Abstract**

Provided is a method of enriching full adenovirus-associated virus (AAV) capsids from a mixture of full AAV capsids and empty AAV capsids, the method comprising providing a sample comprising a mixture of full AAV capsids and empty AAV capsids, subjecting the sample to anion exchange chromatography comprising an elution buffer comprising an equilibration buffer and a salt-containing buffer in an initial ratio that provides an initial conductivity, and changing the ratio of the equilibration buffer and the salt-containing buffer to provide a step gradient conductivity increase of about 0.5-2.0 mS/cm in each step to elute empty AAV capsids and to provide a fluid enriched with full AAV capsids.

## Description

### BACKGROUND OF THE INVENTION

Adenovirus-associated viruses (AAV) are small viruses with a genome of single stranded DNA that can be used as vectors for gene therapy. During the manufacturing of AAV vectors, incomplete particles that lack the desired DNA are produced. The presence of empty AAV particles in a sample increase the dose required for gene therapy and can cause an undesired immunological response. Current methods of separating empty and full AAV particles include density gradient ultracentrifugation and column chromatography. However, such methods are difficult to operate on a large scale with improved separation.

Thus, there remains a need for improved separation of empty and full AAV particles to provide a consistent, higher quality therapeutic agent for gene therapy.

### BRIEF SUMMARY OF THE INVENTION

The invention provides a method of enriching full adenovirus-associated virus (AAV) capsids from a mixture of full AAV capsids and empty AAV capsids. The method comprises providing a sample comprising a mixture of full AAV capsids and empty AAV capsids, subjecting the sample to anion exchange chromatography with an elution buffer comprising an equilibration buffer and a salt-containing buffer in an initial ratio that provides an initial conductivity in the range of about 0.5 mS/cm to about 10 mS/cm, and changing the ratio of the equilibration buffer and the salt-containing buffer to provide a step gradient conductivity increase of about 0.5-2.0 mS/cm in each step to elute empty AAV capsids and to provide a fluid enriched with full AAV capsids.

The method provides advantages over known chromatographic separation methods, including, for example, linear gradients, to produce a product (fluid) with a consistently enriched ratio of full AAV capsids.

### BRIEF DESCRIPTION OF THE SEVERAL VIEWS OF THE DRAWINGS

FIG. 1 is a graph of the separation of empty and full AAV5 particles using a 1 mS/cm step gradient anion-exchange column chromatography method with a positively charged membrane column.
FIG. 2 is a chromatogram of separated empty and full AAV5 particles above an aligned table containing data on the genome copy to capsid ratio for each peak generated, indicating full AAV5 particles are found in later peaks (peaks 3 and 4).
FIG. 3 is a chromatogram of the separation of empty and full AAV5 particles using a 1 mS/cm step gradient anion-exchange column chromatography method with another positively charged membrane column.
FIG. 4 is a chromatogram of the separation of empty and full AAV5 particles using a 1 mS/cm step gradient anion-exchange column chromatography method with a positively charged monolith column.
FIG. 5 shows the peaks generated using the empty AAV5 standard with a positively charged membrane column. Empty AAV5 particles have a higher absorbance at 280 nm compared to 260 nm while eluting at conductivities around 10 mS/cm and 11 mS/cm.
FIG. 6 shows the peaks generated using the full AAV5 standard with a positively charged membrane column. Full particles have equivalent absorbance intensities at both 280 nm and 260 nm while eluting at conductivities around 11 - 13 mS/cm.
FIG. 7 shows the overlay of empty (FIG. 5) and full (FIG. 6) AAV5 standard separation chromatograms.
FIG. 8 illustrates the failed separation of empty and full AAV5 particles using a linear gradient anion-exchange column chromatography method with a positively charged membrane column.
FIG. 9 illustrates the failed separation of empty and full AAV5 particles using a linear gradient anion-exchange column chromatography method with a positively charged monolith column.

### DETAILED DESCRIPTION OF THE INVENTION

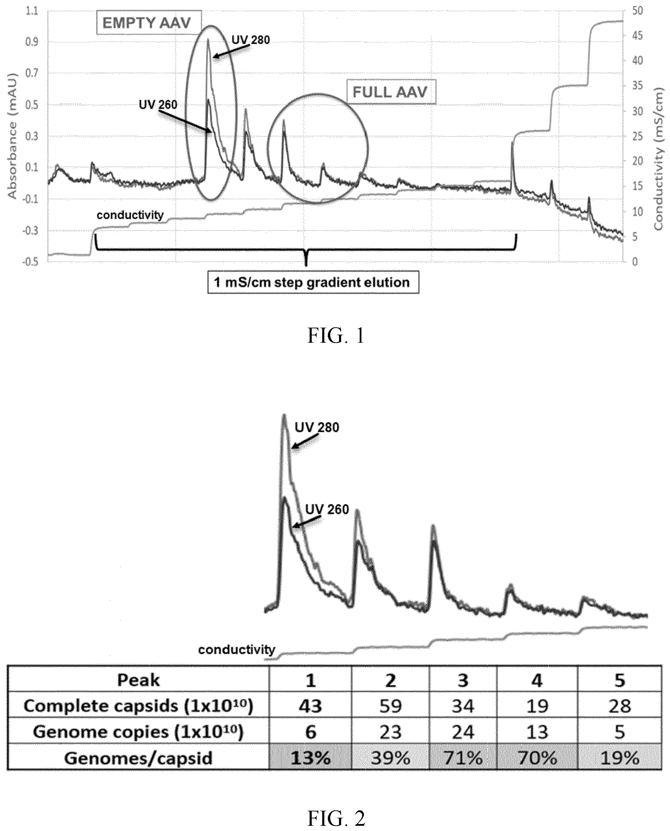

The invention is directed to an anion exchange chromatographic method for the separation of empty and full adenovirus-associated virus (AAV) capsids from a heterogeneous sample. The invention is predicated, at least in part, on the discovery that empty AAV particles elute at lower conductivities than full AAV particles. In particular, full AAV particles have a larger UV absorbance at 260 nm than empty AAV particles (FIG. 1). This separation can also be observed by comparing the UV absorbance at 280 nm and 260 nm, such that empty AAV particles generate a higher 280/260 signal ratio, while full AAV particles generate a 280/260 signal ratio close to 1. In addition to providing improved separation of empty and full AAV particles, this method allows for scaling with manufacturing needs compared to known methods, such as ultra-centrifugation. Other benefits of the inventive method include, for example, rapid processing, use of samples that are serotype-independent, and use of various type of chromatographic media and buffer compositions.

In particular, the invention provides a method of enriching full adenovirus-associated virus (AAV) capsids from a mixture of full AAV capsids and empty AAV capsids, the method comprising
providing a sample comprising a mixture of full AAV capsids and empty AAV capsids,
subjecting the sample to anion exchange chromatography comprising an elution buffer comprising an equilibration buffer and a salt-containing buffer in an initial ratio that provides an initial conductivity in the range of about 0.5 mS/cm to about 10 mS/cm, and
changing the ratio of the equilibration buffer and the salt-containing buffer to provide a step gradient conductivity increase of about 0.5-2.0 mS/cm in each step to elute empty AAV capsids and to provide a fluid enriched with full AAV capsids.

The method is suitable for any serotype, pseudotype, and/or variant of AAV capsids. Suitable serotypes include 1, 2, 3, 4, 5, 6, 7, 8, 9, and combinations thereof. In certain embodiments, the sample comprises AAV serotype 2 (AAV2), AAV serotype 5 (AAV5), or a combination thereof. Psuedotyped AAV particles also can be used, in which a genome of a certain type is disposed within a capsid of a different serotype. For example, the sample can comprise AAV2/5, which includes a genome of serotype 2 disposed in a capsid of serotype 5.

The sample to be separated can have any suitable concentration. For example, the sample can contain at least about 1x10¹¹ capsids/mL (e.g., at least about 1.5x10¹¹ capsids/mL, at least about 1x10¹² capsids/mL, at least about 1.5x10¹² capsids/mL, at least about 1x10¹³ capsids/mL) of chromatograph medium. Preferably, the starting viral load is at least about 1x10¹² capsids/mL of chromatography medium.

The anion exchange chromatography can use any suitable separation method, including an ion exchange membrane (e.g., a positively charged membrane), an ion exchange resin (e.g., a positively charged resin), or a monolith. In some embodiments, subjecting the sample to anion exchange chromatography comprises contacting the sample and a positively charged microporous membrane. The chromatography medium for anionic chromatography can be, for example, MUSTANG^{™} Q XT ACRODISC^{™} (Pall, Port Washington, NY) CIMac^{™} (BIA Separations, Slovenia), POROS HQ (ThermoFisher, Waltham, MA), or POROS XQ (ThermoFisher, Waltham, MA).

In certain embodiments, a final step of the separation method provides an electrical conductivity of about 20 mS/cm. The initial conductivity is any desired value and will depend on the serotype of the AAV particles and buffer compositions. Typically the initial conductivity ranges from 0.5 mS/cm or more to 10 mS/cm or less (e.g., about 0.5 mS/cm, about 0.75 mS/cm, about 1 mS/cm, about 1.5 mS/cm, about 2 mS/cm, about 2.5 mS/cm, about 3 mS/cm, about 3.5 mS/cm, about 4 mS/cm, about 4.5 mS/cm, about 5 mS/cm, about 5.5 mS/cm, about 6 mS/cm, about 6.5 mS/cm, about 7 mS/cm, about 7.5 mS/cm, about 8 mS/cm, about 8.5 mS/cm, about 9 mS/cm, about 9.5 mS/cm, or about 10 mS/cm). For example, the initial conductivity can be about 1 mS/cm, and the conductivity increases with each step until about 20 mS/cm is reached. In general, the step gradient includes incremental changes in conductivity that typically range about 0.5-2.0 mS/cm with each increment. The number of increments to provide the final conductivity will vary depending on the size of the electrical conductivity increments. Preferably, each increment is each about 1 mS/cm. Increments within the method can be the same length or different length. In any of the embodiments described herein, the gradient steps are of a length that allow the ultraviolet (UV) intensities to be within 25% (e.g., within 20%, within 15%) of the baseline signal at the end of the step. Preferably, the UV intensities are within 20% of the baseline signal at the end of the step.

The separation method requires a mixture of an elution buffer comprising an equilibration buffer and a salt-containing buffer. The elution buffer, including the equilibration buffer and salt-containing buffer, can have any suitable pH. Preferably, the pH of the elution buffer, including the pH of the equilibration buffer and the pH of the salt-containing buffer, is basic (e.g., pH greater than 7). In some embodiments, the pH is 7.5 or more, 8 or more, 8.5 or more, 9 or more, 9.5 or more, or 10 or more. In some preferred embodiments, the pH is about 7-10, about 7-9, about 7.5-10.5, about 8-10.5, about 8-10, about 8.5-9.5, or about 9.

The equilibration buffer is any suitable buffer with a conductivity of about 15 mS/cm or less (e.g., about 12 mS/cm or less, about 10 mS/cm or less, about 8 mS/cm or less, about 6 mS/cm or less, about 5 mS/cm or less, about 4 mS/cm or less, about 3 mS/cm or less, about 2 mS/cm or less, about 1 mS/cm). In some embodiments, the equilibration buffer has a conductivity of about 1 mS/cm.

In general, the equilibration buffer will have an operating range with a basic pH (e.g., at least 7), as described herein. The equilibration buffer can be, for example, bis-tris propane (BTP), tris(hydroxymethyl)aminomethane (TRIS), TRIS-Cl, TRIS-HCl, bis-6TRIS propane, ammonium acetate, 2-[[1,3-dihydroxy-2-(hydroxymethyl)propan-2-yl]amino]ethanesulfonic acid (TES), 2-[4-(2-hydroxyethyl)piperazin-1-yl]ethanesulfonic acid (HEPES), 3-(*N,N*-bis[2-hydroxyethyl]amino)-2-hydroxypropanesulfonic acid (DIPSO), 4-(N-morpholino)butanesulfonic acid (MOBS), 4-(2-hydroxyethyl)piperazine-1-(2-hydroxypropanesulfonic acid) hydrate (HEPPSO), piperazine-N,N'-bis(2-hydroxypropanesulfonic acid) (POPSO), 4-(2-hydroxyethyl)-1-piperazinepropanesulfonic acid (EPPS), N,N-bis(2-hydroxyethyl)glycine (bicine), N-[tris(hydroxymethyl)methyl]glycine (tricine), diglycine (gly-gly), N-(2-hydroxyethyl)piperazine-N'-(4-butanesulfonic acid) (HEPBS), 3-{[1,3-dihydroxy-2-(hydroxymethyl)propan-2-yl]amino}propane-1-sulfonic acid (TAPS), 2-amino-2-methyl-1,3-propanediol (AMPD), N-[tris(hydroxymethyl)methyl]-3-aminopropanesulfonic acid (TAPS), N-(1,1-dimethyl-2-hydroxyethyl)-3-amino-2-hydroxypropanesulfonic acid (AMPSO), 2-(cyclohexylamino)ethanesulfonic acid (CHES), 3-(cyclohexylamino)-2-hydroxy-1-propanesulfonic acid (CAPSO), 3-(cyclohexylamino)-1-propanesulfonic acid (CAPS), 4-(cyclohexylamino)-1-butanesulfonic acid (CABS), or 2-amino-2-methyl-1-propanol (AMP).

The salt-containing buffer (e.g., a high salt-containing buffer) is any suitable equilibration buffer, such as those described herein, but with the addition of an inorganic salt. In preferred embodiments, the salt-containing buffer is the same as the equilibration buffer (e.g., BTP). In certain embodiments, the salt is present in the buffer in an amount to provide a conductivity of about 15 mS/cm or more (e.g., about 20 mS/cm or more, about 30 mS/cm or more, about 40 mS/cm or more, about 50 mS/cm or more, about 60 mS/cm or more, about 65 mS/cm or more, about 70 mS/cm or more, about 75 mS/cm or more, about 80 mS/cm or more, or about 85 mS/cm or more). In some preferred embodiments, the electrical conductivity of the salt-containing buffer is about 50 mS/cm or more, preferably about 80 mS/cm or more. Generally, the concentration of salt in the buffer is at least 100 mM (e.g., at least 200 mM, at least 300 mM, at least 500 mM, at least 600 mM, at least 800 mM, at least 900 mM, at least 1 M, at least 1.1 M, at least 1.2 M, at least 1.3 M, at least 1.5 M, at least 1.6 M, at least 1.8 M, at least 1.9 M). In some embodiments, the concentration of salt in the buffer is 2 M or less (e.g., 1.9 M or less, 1.8 M or less, 1.6 M or less, 1.5 M or less, 1.3 M or less, 1.2 M or less, 1.1 M or less, 1 M or less, 900 mM or less, 800 mM or less, 600 mM or less, 500 mM or less, 300 mM or less, or 200 mM as less). Any two of the foregoing endpoints can be used to define a close-ended range, or a single endpoint can be used to define an open-ended range. For example, the salt concentration can be 200 mM to 2 M, 500 mM to 1.5 M, 900 mM to 1.2 M, or about 1 M.

The salt is any inorganic salt, such as any salt containing a cation of a Group I metal (lithium, sodium, potassium, rubidium, or cesium), a Group II metal (beryllium, magnesium, calcium, strontium, or barium), ammonium, or aluminum. The counter anion can be a halide, carbonate, bicarbonate, sulfate, thiosulfate, phosphate, nitrate, nitrite, acetate, bromate, chlorate, iodate, etc. Specific examples of salt include lithium bromide, lithium chloride, lithium iodate, lithium iodide, lithium hydroxide, lithium sulfate, lithium phosphate, sodium bromide, sodium chloride, sodium acetate, sodium bicarbonate, sodium bisulfate, sodium bromate, sodium chlorate, sodium hydrosulfide, sodium hydroxide, sodium hypophosphite, sodium iodate, sodium iodide, potassium acetate, potassium bicarbonate, potassium bromate, potassium bromide, potassium chloride, potassium carbonate, potassium chlorate, potassium hydroxide, potassium iodide, potassium phosphate, potassium thiosulfate, rubidium bromide, rubidium chloride, rubidium fluoride, rubidium iodide, rubidium nitrate, rubidium sulfate, cesium bromide, cesium chloride, cesium carbonate, cesium nitrate, beryllium nitrate, beryllium sulfate, magnesium acetate, magnesium bromide, magnesium chloride, magnesium iodate, magnesium iodide, magnesium nitrate, magnesium phosphate, magnesium sulfate, calcium acetate, calcium bromide, calcium chloride, calcium iodide, calcium iodate, calcium nitrite, calcium nitrate, calcium phosphate, calcium sulfate, strontium bromide, strontium chloride, strontium hydrogen phosphate, strontium iodide, strontium nitrate, strontium sulfate, barium acetate, barium bromide, barium chloride, barium iodide, barium nitrate, barium phosphate, barium sulfate, barium thiosulfate, ammonium acetate, ammonium bicarbonate, ammonium bromide, ammonium chloride, ammonium nitrate, aluminum chloride, aluminum phosphate, and any combination thereof. In some embodiments, the salt is a Group I-halide or Group I-acetate salt, such as sodium chloride, sodium acetate, potassium chloride, or potassium acetate.

The concentrations of equilibration buffer and salt-containing buffer are used in any suitable concentration. In certain embodiments, the equilibration buffer and salt-containing buffer are each used in a concentration of at least 5 mM (e.g., at least 10 mM, at least 15 mM, at least 20 mM, at least 25 mM, at least 30 mM, at least 35 mM, at least 40 mM, at least 45 mM, or at least 50 mM). Typically, the equilibration buffer and salt-containing buffer are each used in a concentration of 100 mM or less (e.g., 90 mM or less, 85 mM or less, 80 mM or less, 75 mM or less, 70 mM or less, 65 mM or less, 60 mM or less, 55 mM or less, 50 mM or less, 45 mM or less, 40 mM or less, 35 mM or less, 30 mM or less, 25 mM or less, or 20 mM or less). Any two of the foregoing endpoints can be used to define a close-ended range, or a single endpoint can be used to define an open-ended range. The concentrations of equilibration buffer and salt-containing buffer typically will be different and will vary based on the desired conductivity level. In some embodiments, the concentrations of equilibration buffer and salt-containing buffer will each be between 10-100 mM, preferably between 20-50 mM.

Incrementally changing the conductivity during the separation method occurs by altering the ratio of the equilibration buffer and salt-containing buffer. Thus, the ratio of buffers in the elution buffer will change in accordance with the desired step gradient increment (e.g., about 1 mS/cm). The mixture of buffers can be premixed or mixed within the chromatography system. The initial ratio of equilibration buffer to salt-containing buffer is any ratio that provides the desired initial conductivity (e.g., about 1 mS/cm), for example an initial ratio within 90:10 to 100:0. In some embodiments, the initial ratio of equilibration buffer to salt-containing buffer is about 98.3:1.7. The final ratio of equilibration buffer to salt-containing buffer is any ratio that provides the desired final conductivity (e.g., about 20 mS/cm), for example a final ratio within 70:30 to 85:15. In some embodiments, the final ratio of equilibration buffer to salt-containing buffer is about 81.5:18.5. In a preferred embodiment, the initial ratio of equilibration buffer to salt-containing buffer is about 98.3:1.7, and the final ratio of equilibration buffer to salt-containing buffer is about 81.5:18.5.

As used herein the term "about" typically refers to ± 1% of a value, ± 5% of a value, or ± 10% of a value.

The elution volume will vary by the sample volume and column size. Each step of the gradient can produce a different volume. When the elution steps are of insufficient volume (e.g., less than 2 volumes of the chromatographic device), the peaks are incompletely eluted within an elution step, and the separation is compromised.

As the separation method proceeds, the resolution of the chromatographic peaks can be monitored by UV wavelengths at 280 nm to monitor protein concentration (empty and full capsids) and 260 nm to monitor DNA concentration (full capsids). In any of the embodiments herein, the fluid from each conductivity step is collected and samples containing protein and DNA, as identified by UV, are further analyzed. Analysis is typically performed via an enzyme-linked immunosorbent assay (ELISA) to calculate the total number of capsids (i.e., protein) in a sample and via polymerase chain reaction (PCR) (e.g., DROPLET DIGITAL^{™} PCT (ddPCR) or quantitative PCR (qPCR)), which can identify the number of genome copies. The ratio of ELISA to PCR can indicate the amount of full capsids, which can be subsequently confirmed by additional analytical methods, such as transmission electron microscopy and/or analytical ultracentrifugation.

The separation method provides a resolution that elutes empty AAV capsids at lower conductivities to provide a fluid enriched with full AAV capsids. The term "enriched" means that the fluid contains more full AAV particles relative to empty AAV particles after performing the inventive method compared to the starting sample. In certain embodiments, the separated sample (fluid) is enriched with at least 50% (e.g., at least 55%, at least 60%, at least 65%, at least 70%, at least 75%, at least 80%, or at least 85%) full AAV capsids relative to empty AAV capsids. Preferably, the method provides an enrichment of at least 70% full AAV relative to empty AAV capsids in the final product (fluid).

The method described herein typically is part of a larger production process. Thus, additional phases of the production process can include, for example, generation of the AAV sample (upstream cell culture), initial filtration of AAV (clarification/tangential flow filtration (TFF)), and/or purification of AAV (e.g., by affinity chromatography). Such steps will precede the anion-exchange chromatography method to separate the empty and full AAV particles using a conductivity gradient elution of the present invention. The inventive separation method can further include initial steps, including equilibration of the column (e.g., membrane), application of the AAV-containing sample, and/or washing of the column (e.g., membrane), followed by the conductivity step gradient.

The invention is further illustrated by the following embodiments.
(Embodiment 1) A method of enriching full adenovirus-associated virus (AAV) capsids from a mixture of full AAV capsids and empty AAV capsids, the method comprising providing a sample comprising a mixture of full AAV capsids and empty AAV capsids, subjecting the sample to anion exchange chromatography comprising an elution buffer comprising an equilibration buffer and a salt-containing buffer in an initial ratio that provides an initial conductivity in the range of about 0.5 mS/cm to about 10 mS/cm, and changing the ratio of the equilibration buffer and the salt-containing buffer to provide a step gradient conductivity increase of about 0.5-2.0 mS/cm in each step to elute empty AAV capsids and to provide a fluid enriched with full AAV capsids.
(Embodiment 2) The method of embodiment (1), wherein subjecting the sample to anion exchange chromatography comprises contacting a positively charged microporous membrane with the sample.
(Embodiment 3) The method of embodiments (1) or (2), wherein a final step provides a conductivity of about 20 mS/cm.
(Embodiment 4) The method of any one of embodiments (1)-(3), wherein the initial ratio of equilibration buffer to salt-containing buffer is about 98.3:1.7.
(Embodiment 5) The method of any one of embodiments (1)-(4), wherein the final ratio of equilibration buffer to salt-containing buffer is about 81.5:18.5.

The following examples further illustrate the invention but, of course, should not be construed as in any way limiting its scope.

### EXAMPLE 1

This example demonstrates a protocol for achieving separation of empty and full AAV particles using a conductivity step gradient. The steps are set forth in Table 1.

**Table 1**

| **Step** | **Buffers used** | **Volume used (Column Volumes)** |
|---|---|---|
| 1 - Equilibration of membrane | Buffer A - Equilibration buffer | 10 |
| 2 - Application of sample | AAV Sample solution | Varies by sample volume |
| 3 - Washing of membrane | Buffer A - Equilibration buffer | 10 |
| 4 - Step Gradients | Buffer A - Equilibration buffer | 10 per step |
| | Buffer B - Salt-containing buffer | |
| | Each step uses a percent mix of A and B to allow an increase of 1-2 mS/cm per step Ex. Initial step could be 98%A, 2%B Following step could be 96%A, 4%B, and so on to final conductivity > 20 mS/cm | |

### EXAMPLE 2

This example describes an AKTA^{™} Avant protocol for achieving separation of empty and full AAV particles.

An affinity-purified AAV sample solution containing empty and full viral particles was obtained and applied to an anion-exchange column chromatography on an automated fast protein liquid chromatography (FPLC) system (PALL MUSTANG^{™} Q ACRODISC^{™} (0.86 mL CV)). The elution buffer comprised:
Equilibration buffer of low conductivity (< 5 mS/cm) at a pH range of 8.5 to 9.5 High salt buffer of higher conductivity (> 80 mS/cm) at a pH range of 8.5 to 9.5

The method used to achieve separation is set forth in Table 2.

**Table 2**

| **Step** | **Buffers used/details** | **Volume used (Column Volumes)** |
|---|---|---|
| 1 - Equilibration of membrane | Buffer A - Equilibration buffer | 10 |
| 2 - Application of sample | AAV Sample solution | Varies by sample volume |
| 3 - Washing of membrane | Buffer A - Equilibration buffer | 10 |
| 4 - Step Gradients | Buffer A - Equilibration buffer | 10 per step |
| | Buffer B - High salt buffer | |
| | Step 1 = 1.7% Buffer B, 98.3% Buffer A | |
| | Step 2 = 2.5% Buffer B, 97.5% Buffer A | |
| | Step 3 = 3.5% Buffer B, 96.5% Buffer A | |
| | Step 4 = 4.4% Buffer B, 95.6% Buffer A | |
| | Step 5 = 5.4%% Buffer B, 94.6% Buffer A | |
| | Step 6 = 6.4% Buffer B, 93.6% Buffer A | |
| | Step 7 = 7.3% Buffer B, 92.7% Buffer A | |
| | Step 8 = 8.3% Buffer B, 91.7% Buffer A | |
| | Step 9 = 9.4% Buffer B, 90.6% Buffer A | |
| | Step 10 = 10.4% Buffer B, 89.6% Buffer A | |
| | Step 11 = 11.4% Buffer B, 88.6% Buffer A | |
| | Step 12 = 12.4% Buffer B, 87.6% Buffer A | |
| | Step 13 = 13.4% Buffer B, 86.6% Buffer A | |
| | Step 14 = 14.4% Buffer B, 85.6% Buffer A | |
| | Step 15 = 15.4% Buffer B, 84.6% Buffer A | |
| | Step 16 = 16.4% Buffer B, 83.6% Buffer A | |
| | Step 17 = 17.5% Buffer B, 82.5% Buffer A | |
| | Step 18 = 18.5% Buffer B, 81.5% Buffer A | |

The separation of empty and full capsids was monitored by absorbance at wavelengths of 280 nm for protein content, and 260 nm for DNA content. The final elutions containing a higher ratio of full AAV capsids were confirmed by performing DROPLET DIGITAL^{™} polymerase chain reaction (ddPCR) (Bio-Rad, Hercules, CA) to measure the number of genome copies present in the eluted viral particles. Capsid enzyme linked immunosorbent assay (ELISA) was used to measure the total number of formed AAV capsids present in the eluted viral particles. FIG. 2 shows the elution peaks generated at higher conductivity steps contained greater amounts of genome copies relative to earlier elution peaks.

### EXAMPLE 3

This example describes a method for separating empty and full AAV particles using anionic chromatography with a membrane.

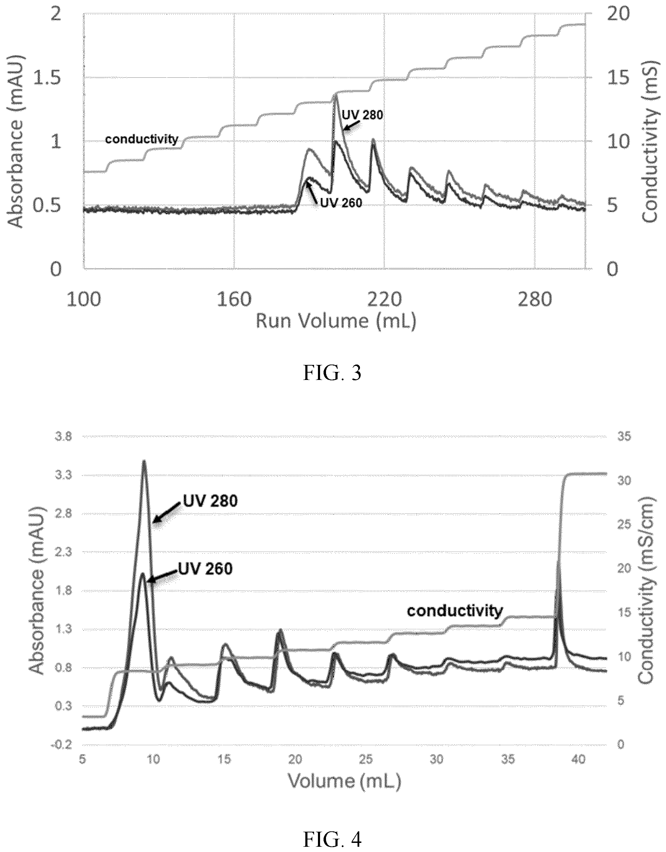

Example 2 was replicated using a 1 mS/cm elution method using a SARTOBIND^{™} Q (Sartorius, France) 1 mL membrane column loaded with a sample comprising AAV5 particles. The results are shown in FIG. 3.

### EXAMPLE 4

This example describes a method for separating empty and full AAV particles using anionic chromatography with a monolith column.

Example 2 was replicated using a 1 mS/cm elution method using a CIMac^{™} (BIA Separations, Slovenia) 0.1 mL monolith column loaded with a sample comprising AAV5 particles. The results are shown in FIG. 4.

### EXAMPLE 5

This example illustrates the distinct chromatographic patterns of empty AAV5 particles versus full AAV5 particles.

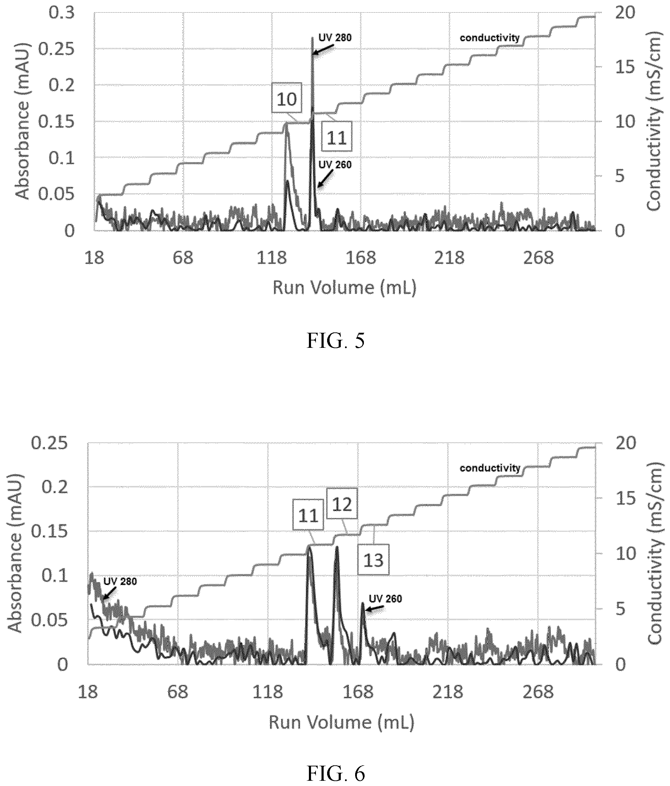

AAV5 empty and full particle standards were obtained via ultracentrifugation, a common industry approach to separate empty and full AAV particles. The conductivity step gradient elution method of the invention was performed on the empty and full AAV5 standards. FIG. 5 shows the peaks generated using the empty AAV5 standard, in which empty AAV particles have a higher absorbance at 280 nm compared to 260 nm while eluting at conductivities around 10 mS/cm and 11 mS/cm. FIG. 6 shows the peaks generated using the full AAV5 standard, in which full particles have equivalent absorbance intensities at both 280 nm and 260 nm while eluting at conductivities around 11 - 13 mS/cm.

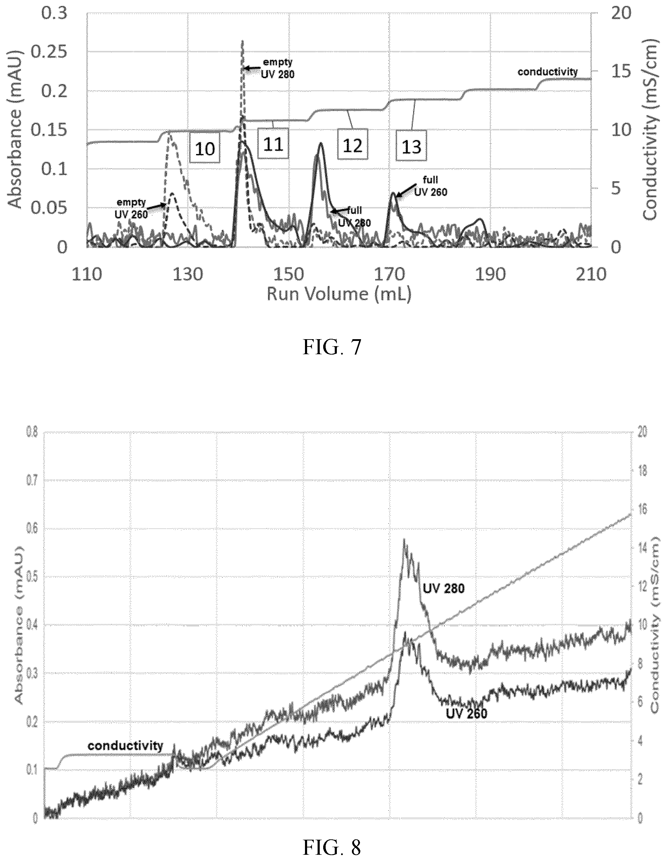

FIG. 7 shows the overlay of empty and full standard separation chromatograms. Empty AAV5 particles elute at lower conductivity compared to full AAV5 particles. These chromatograms demonstrate that separation of AAV5 empty and full particles can be achieved using this conductivity step approach.

### COMPARATIVE EXAMPLE 1

This example describes a method of separating empty and full AAV particles using a linear gradient anion-exchange column chromatography method.

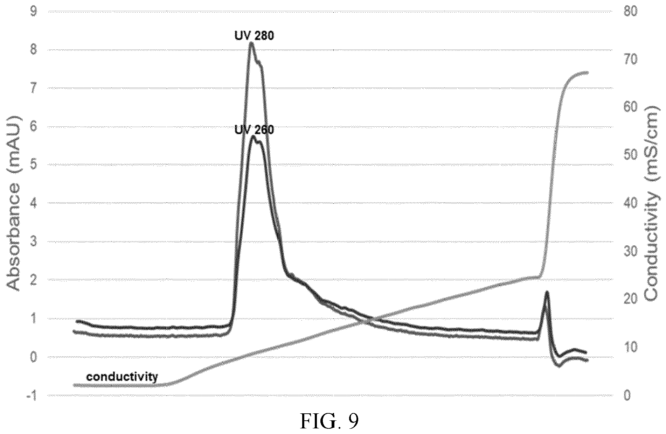

The method used a linear salt gradient elution (0 - 200 mM) for general AAV capsid separation of empty and full AAV5 particles when using either (a) MUSTANG^{™} Q XT 0.86mL ACRODISC^{™} (Pall, Port Washington, NY) (FIG. 8) or (b) a 0.1 mL Analytical CIMac^{™} monolith column (BIA Separations, Slovenia) (FIG. 9). As seen in FIGs. 8 and 9, a commonly-used linear gradient elution did not show separation of empty and full AAV5 viral vectors on either PALL MUSTANG^{™} Q membrane or CIMac^{™} monolith (BIA Separations).

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A method of enriching full adenovirus-associated virus (AAV) capsids from a mixture of full AAV capsids and empty AAV capsids, the method comprising
providing a sample comprising a mixture of full AAV capsids and empty AAV capsids,
subjecting the sample to anion exchange chromatography comprising an elution buffer comprising an equilibration buffer and a salt-containing buffer in an initial ratio that provides an initial conductivity in the range of about 0.5 mS/cm to about 10 mS/cm, and
changing the ratio of the equilibration buffer and the salt-containing buffer to provide a step gradient conductivity increase of about 0.5-2.0 mS/cm in each step to elute empty AAV capsids and to provide a fluid enriched with full AAV capsids.

2. The method of claim 1, wherein subjecting the sample to anion exchange chromatography comprises contacting a positively charged microporous membrane with the sample.

3. The method of claim 1 or 2, wherein a final step provides a conductivity of about 20 mS/cm.

4. The method of any one of claims 1-3, wherein the initial ratio of equilibration buffer to salt-containing buffer is about 98.3:1.7.

5. The method of any one of claims 1-4, wherein the final ratio of equilibration buffer to salt-containing buffer is about 81.5:18.5.
